(19) Europäisches Patentamt
European Patent Office
Office européen des brevets

(11) EP 4 495 144 A1

(12) EUROPEAN PATENT APPLICATION
published in accordance with Art. 153(4) EPC

(43) Date of publication:
**22.01.2025 Bulletin 2025/04**

(21) Application number: **23741246.5**

(22) Date of filing: **01.03.2023**

(51) International Patent Classification (IPC):
***C08F 2/48*** *(2006.01)* ***C07D 295/104*** *(2006.01)*
***C07D 487/04*** *(2006.01)* ***C07D 251/30*** *(2006.01)*
***C09D 4/02*** *(2006.01)* ***C09D 175/14*** *(2006.01)*
***C09D 167/06*** *(2006.01)*

(52) Cooperative Patent Classification (CPC):
(C-Sets available)
**C08K 5/378; C09D 4/06;** Y02P 20/10 (Cont.)

(86) International application number:
**PCT/CN2023/079014**

(87) International publication number:
**WO 2023/174056 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.03.2022 CN 202210246991**

(71) Applicant: **Inner Mongolia Yangfan New Material Co., Ltd.**
**Alxa, Inner Mongolia 750399 (CN)**

(72) Inventors:
- **JIN, Ming**
  **Alxa, Inner Mongolia 750399 (CN)**
- **LIAO, Wen**
  **Alxa, Inner Mongolia 750399 (CN)**
- **GUO, Feng**
  **Alxa, Inner Mongolia 750399 (CN)**
- **LI, Jingdong**
  **Alxa, Inner Mongolia 750399 (CN)**
- **YANG, Chengshuang**
  **Alxa, Inner Mongolia 750399 (CN)**
- **LIN, Lidong**
  **Alxa, Inner Mongolia 750399 (CN)**
- **FAN, Bin**
  **Alxa, Inner Mongolia 750399 (CN)**

(74) Representative: **Chung, Hoi Kan**
**Mandarin IP Limited**
**7 Cherry Trees**
**Great Shelford**
**Cambridge CB22 5XA (GB)**

# (54) MULTIFUNCTIONAL MACROMOLECULAR PHOTOINITIATOR CONTAINING A-AMINOKETONE, AND PREPARATION METHOD THEREFOR AND USE THEREOF

(57) An α-aminoketone-containing polyfunctionalized macromolecular photoinitiator, which is prepared from a double-bond-containing α-aminoketone micromolecular photoinitiator A and a polythiol compound B through thiol-ene click reaction. A preparation of the α-aminoketone-containing polyfunctionalized macromolecular photoinitiator and an application of the α-aminoketone-containing polyfunctionalized macromolecular photoinitiator in the radiation curing are also provided.

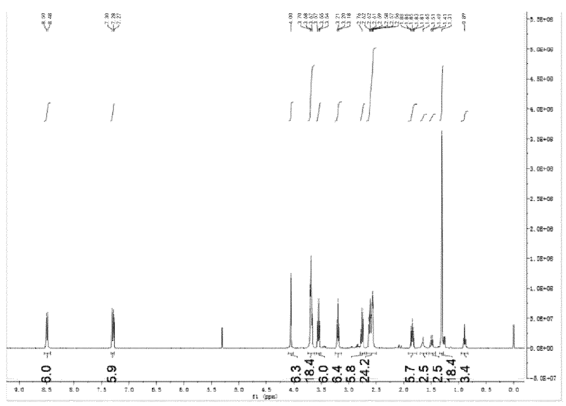


FIG.1

EP 4 495 144 A1

(52) Cooperative Patent Classification (CPC): (Cont.)

C-Sets
**C09D 4/00, C08F 222/102;**
**C09D 4/00, C08F 222/103**

## Description

### CROSS-REFERENCE TO RELATED APPLICATIONS

This application is a continuation of International Patent Application No. PCT/CN2023/079014, filed on March 1, 2023, which claims the benefit of priority from Chinese Patent Application No. 202210246991.6, filed on March 14, 2022. The content of the aforementioned application, including any intervening amendments thereto, is incorporated herein by reference in its entirety.

### TECHNICAL FIELD

This application relates to organic chemicals, and more specifically to a polyfunctionalized macromolecular photoinitiator containing $\alpha$-aminoketone, and a preparation and application thereof.

### BACKGROUND

Light curing is light-induced polymerization of liquid photosensitive resins into solids, which is characterized by high reaction efficiency, less time and energy consumption and excellent environmental friendliness, and thus has been widely used in the adhesives, light-curable coatings and inks, photoresists, 3D microstructures, and biopharmaceuticals. The photoinitiator is the key factor to control the whole photopolymerization process, and its activity will directly affect the light curing rate, the curing degree, and performances of the end products. Especially for the products, which are in direct contact with human body, such as the packaging of food and drug and children's toys, there are higher requirements for migration and odor of residual photoinitiators. Therefore, extensive researches have been conducted to design and develop a low-toxicity, low-migration, and low-odor photoinitiator.

Photoinitiators can be divided into free-radical and cationic photoinitiators. Regarding the free-radical light curing, the photoinitiators will undergo rapid decomposition under the exposure to light to generate reactive radicals, so as to initiate the chain cross-linking polymerization of a light-curable resin with double bonds and a reactive diluent.

The $\alpha$-aminoketone photoinitiators stand out from a wide variety of photoinitiators due to its good initiation effect, simple structure, and easy synthesis, represented by photoinitiator 907:

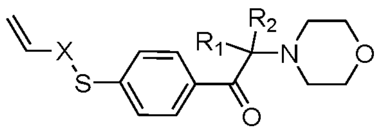

.

The photoinitiator 907 has been widely used in the light curing process in the fields of ink, paint, and cosmetics due to its good compatibility with other materials. However, the photoinitiator 907 is toxic, and is thus limited for the application in packaging of food, pharmaceuticals, and hygiene products. For example, the photoinitiator 907 can initiate the rapid curing of an acrylate system, and will be partially physically held by the acrylate crosslinking network after the light curing process, and the residual photoinitiator will undergo molecular migration, penetration or extraction once being exposed to hot water, solvents, or grease, thereby limiting the use of the photoinitiator 907 in the fields with high requirements for biological toxicity.

In view of the deficiencies in the prior art, this application provides a modified photoinitiator, and a preparation and application thereof.

### SUMMARY

In view of the deficiencies in the prior art, this application provides a polyfunctionalized macromolecular photoinitiator containing $\alpha$-aminoketone and a preparation and application thereof. The photoinitiator I provided herein can be used as a substitute for the conventional photoinitiator 907, and has a wider application range than the photoinitiator 907.

Technical solutions of this application are described as follows.

In a first aspect, this application provides an $\alpha$-aminoketone-containing polyfunctionalized macromolecular photoinitiator, wherein the $\alpha$-aminoketone-containing polyfunctionalized macromolecular photoinitiator is prepared from a double-bond-containing $\alpha$-aminoketone photoinitiator A and a polythiol compound B through thiol-ene click reaction.

In an embodiment, the $\alpha$-aminoketone-containing polyfunctionalized macromolecular photoinitiator has a molecular weight of 1000 or more.

In an embodiment, the double-bond-containing $\alpha$-aminoketone photoinitiator A is expressed as:

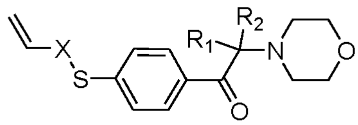

;

wherein $R_1$ and $R_2$ are each independently $C_1$-$C_{18}$ alkyl, $-(CH_2)_r-C_3-C_6$ cycloalkyl group or $-(CH_2)_r$-phenyl, wherein r is 0, 1, 2, 3, or 4; and

X is an unsubstituted alkyl, a substituted alkyl with at least one $-CH_2-$ each independently substituted with -O-, -CO-, -COO-, -OCO-, or $-O-CH_2-CH(OH)-CH_2-O-$, or absent.

In an embodiment, both $R_1$ and $R_2$ are methyl when X is absent.

In an embodiment, the polythiol compound B is a trithiol or a tetrathiol.

In an embodiment, the polythiol compound B is selected from the group consisting of:

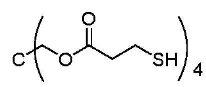

**B1** ,

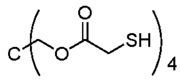

**B2** ,

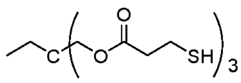

**B3** ,

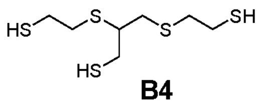

**B4** ,

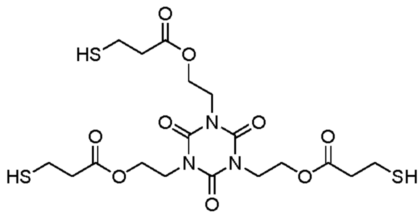

**B5** ,

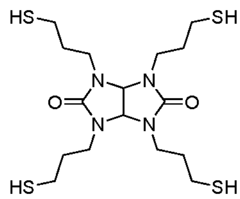

**B6** ,

and a combination thereof.

In an embodiment, the α-aminoketone-containing polyfunctionalized macromolecular photoinitiator is selected from the group consisting of:

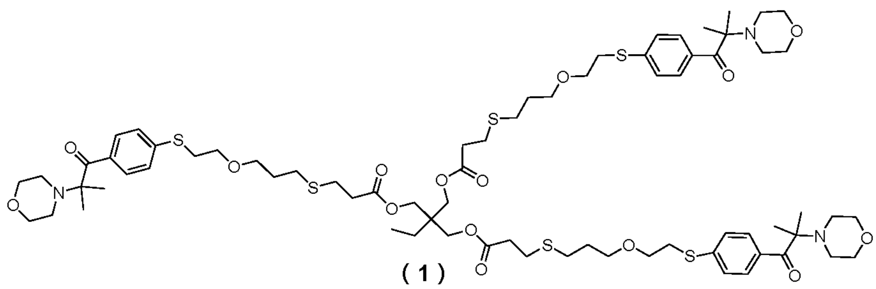

(1)

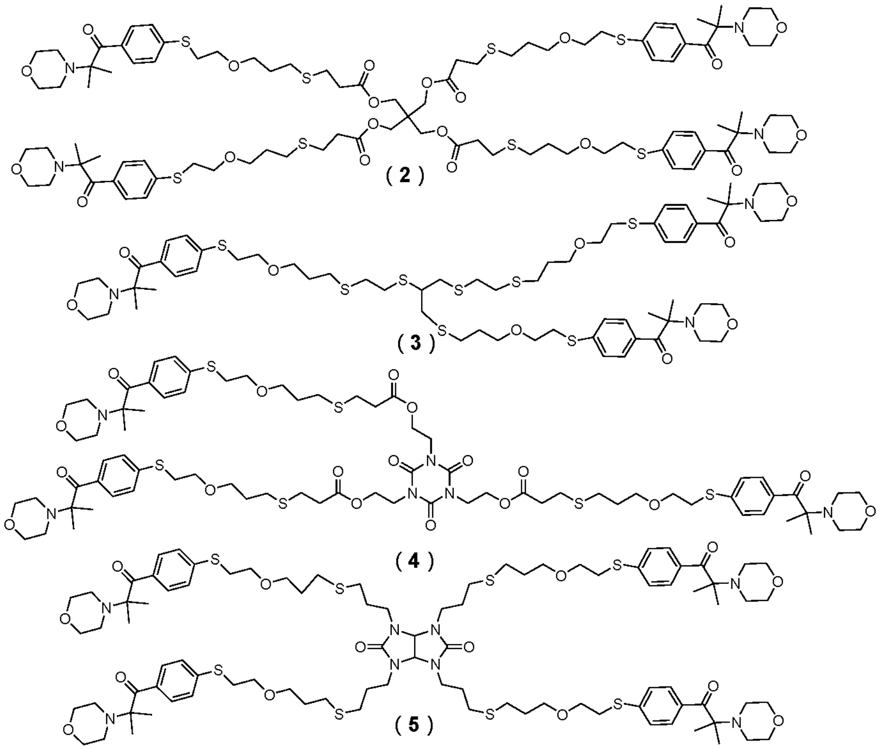

(2)

(3)

(4)

(5)

.

In a second aspect, this application provides a method for preparing the α-aminoketone-containing polyfunctionalized macromolecular photoinitiator, comprising:

(a) mixing the double-bond-containing α-aminoketone photoinitiator A and the polythiol compound B to obtain a mixture; wherein a molar ratio of a carbon-carbon double bond in the double-bond-containing α-aminoketone photoinitiator A to a thiol group in the polythiol compound B is 1:1;
(b) adding an azodiisobutyronitrile (AIBN) thermal initiator or a benzoyl peroxide thermal initiator into the mixture followed by stirring under nitrogen atmosphere and reaction at 60~100°C for 2~12 h to obtain a reaction solution, wherein the AIBN thermal initiator or the benzoyl peroxide thermal initiator is 0.5%-3% of a total weight of the mixture, and whether the reaction is completed or not is determined by monitoring an absorption peak of thiol by infrared spectroscopy, and when the absorption peak of thiol is absent, it indicates completion of the reaction; and
(c) subjecting the reaction solution to recrystallization to obtain the α-aminoketone-containing polyfunctionalized macromolecular photoinitiator.

In a third aspect, this application provides use of the α-aminoketone-containing polyfunctionalized macromolecular photoinitiator in a photoinitiator composition for radiation curing or as an intermediate, raw material or reagent in the chemical synthesis.

In an embodiment, an excitation light source of the photoinitiator composition is ultraviolet light, visible light, or a combination thereof.

In an embodiment, the photoinitiator composition comprises 0.01~30 parts by weight of the α-aminoketone-containing polyfunctionalized macromolecular photoinitiator and 100 parts by weight of a double bond-containing unsaturated compound.

In an embodiment, the photoinitiator composition comprises 0.5~10 parts by weight of the α-aminoketone-containing polyfunctionalized macromolecular photoinitiator and 100 parts by weight of the double bond-containing unsaturated compound.

In an embodiment, the double bond-containing unsaturated compound is a compound or mixture whose double bonds are cross-linked by free-radical polymerization reaction; and
the double bond-containing unsaturated compound is selected from the group consisting of a monomer, an oligomer, a prepolymer, a combination thereof, a copolymer thereof and an aqueous dispersion thereof.

In an embodiment, the photoinitiator composition further includes an auxiliary agent, and a weight ratio of the double bond-containing unsaturated compound to the auxiliary agent is 100: 0 ~ 4.5.

In an embodiment, the photoinitiator composition is used through the following steps:

(1) mixing the double bond-containing unsaturated compound, the photoinitiator composition, and the auxiliary agent with a mass ratio of 100: 0.5-1: 0~4.5 to form a polymerization system;
(2) irradiating the polymerization system using the excitation light source of the photoinitiator composition; and
(3) calculating the polymerization conversion by the change of the characteristic peaks using the spectral analysis method.

In an embodiment, the auxiliary agent is selected from, but is not limited to, the group consisting of an inorganic filler, an organic filler, a colorant, a solvent, an additive, and a combination thereof.
In an embodiment, the additive is a UV absorber, a light stabilizer, a flame retardant, a leveling agent, or a defoamer. The colorant is a pigment or a dye.
In an embodiment, the monomer is selected from the group consisting of (meth)acrylate, acrolein, olefin, conjugated diolefin, styrene, maleic anhydride, fumaric acid anhydride, vinyl acetate, vinylpyrrolidone, vinylimidazole, acrylic acid, acrylic acid derivatives (e.g., acrylamide), methacrylic acid, methacrylic acid derivatives (e.g., methacrylamide) vinyl halide and vinylidene halide.
In an embodiment, the prepolymer or oligomer is selected from the group consisting of (meth)acrylate copolymers with (meth) Acryloyl functional groups, polyurethane formate (meth)acrylate, polyester (meth)acrylate, unsaturated polyester, polyether (meth)acrylates, siloxane (meth)acrylate, epoxy resin (meth)acrylate and water-soluble and water-dispersible analogues thereof.
Compared to the prior art, this application has the following beneficial effects.

(1) The photoinitiator I obtained in the application has almost the same photoinitiability with the conventional photoinitiator 907. The photoinitiator I can replace the photoinitiator 907. The photoinitiator I has a wider application range than the photoinitiator 907. The photoinitiator I can be applied to fields with high requirements for biological toxicity, such as food and pharmaceutical packaging coatings, and contact biomedical materials.
(2) 2-morpholinyl-2-methylpropiophenone derivatives connect to double bond groups through ether or ester bonds, and react with polythiol to prepare photoinitiator I through thiol-ene click reaction. The conversion rate of the reactants is close to 100%. The preparation method is simple, environmentally friendly, easy to obtain raw materials, and easy to realize industrial production.
(3) The photoinitiator I has a molecular weight of 1000 or more. Compared with the photoinitiator 907, the photoinitiator I and residues after being photolyzed will have significantly lower migration and odor when initiating the curing of the light-curing system, making the photoinitiator I suitable for the fields with high requirements for biological toxicity.

## BRIEF DESCRIPTION OF THE DRAWINGS

FIG. 1 shows a $^1H$ NMR spectrum of photoinitiator 1 in $CDCl_3$ according to an embodiment of the disclosure;
FIG. 2 shows a $^1H$ NMR spectrum of photoinitiator 3 in $CDCl_3$ according to an embodiment of the disclosure;
FIG. 3 shows photopolymerization kinetic curves of trimethylolpropane triacrylate (TMPTA) monomers initiated respectively by the photoinitiator 1 and photoinitiator 907 of the same weight;
FIG. 4 shows Photo-differential scanning calorimetry (DSC) curves of TMPTA monomers initiated respectively by the photoinitiator 1 and the photoinitiator 907 in the same number of moles;
FIG. 5 shows DSC curves of the photoinitiator 1 and the photoinitiator 907; and
FIG. 6 shows a $^1H$ NMR spectrum of a double-bond-containing $\alpha$-aminoketone photoinitiator A prepared in Example 2 of the present disclosure in $CDCl_3$.

## DETAILED DESCRIPTION OF EMBODIMENTS

The double-bond-containing $\alpha$-aminoketone micromolecular photoinitiator A is expressed as:

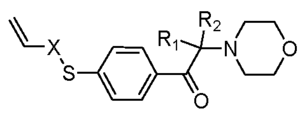
,

and when X is an alkyl group, the photoinitiator A is commercially available. The polythiol compound B is also commercially available.
The disclosure will be further described below in conjunction with the examples.

Example 1 Preparation of **double-bond-containing α-aminoketone** micromolecular photoinitiator A

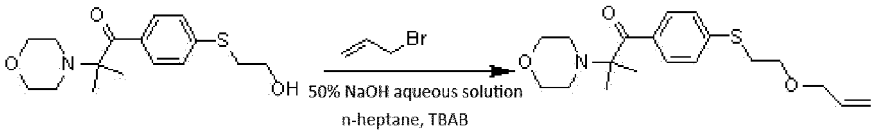


To a jacketed reaction flask were added 30.9 g of 2-morpholinyl-4'-(2-hydroxyethylthio)-2-methylpropiophenone (0.1 mol) and 300 mL of n-heptane, to which 12 g of a NaOH aqueous solution with a mass concentration of 50% and 0.3 g of tetrabutylammonium bromide (TBAB) were dropwise added. The cooling water was supplied, and the reaction mixture was dropwise added with 50 mL of a solution of allyl bromide (0.12 mol) in n-heptane in the nitrogen atmosphere and reacted at 25°C, where the temperature of the reaction system would rise immediately after the solution was dropwise added, and was required to be controlled to below 30°C; the dropwise addition lasted for about 30 minutes, and the reaction was performed under stirring at room temperature for 5 h. After the reaction was monitored by thin layer chromatography (TLC) to be completed, the reaction mixture was washed twice with deionized water, dried with anhydrous sodium sulfate, and subjected to rotary evaporation to remove n-heptane solvent and excess allyl bromide and drying in a vacuum oven for 24 h to obtain a viscous light-yellow sample for direct use.

The yield of the double-bond-containing α-aminoketone micromolecular photoinitiator A was 98%. MS ($C_{19}H_{27}NO_3S$): m/e: 349.49; and experimental result: 350.50 ($M+H^+$).

**Example 2 Preparation of double-bond-containing α-aminoketone photoinitiator A**

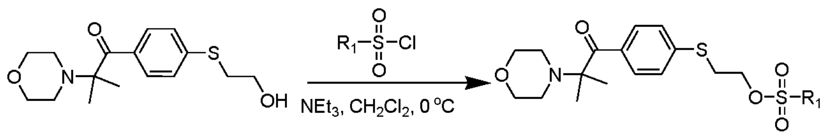


To a jacketed reaction flask were added 30.9 g of 2-morpholinyl-4'-(2-hydroxyethylthio)-2-methylpropiophenone (0.1 mol) and 300 mL of anhydrous dichloromethane, to which 12.2 g of triethylamine (0.12 mol) was added. The cooling water was supplied, and the reaction mixture was dropwise added with 50 mL of methylsufonyl chloride (0.105 mol) or 50 mL of dichloromethane solution of paratoluensulfonyl chloride (0.105 mol) in the nitrogen atmosphere, and reacted at 0°C. The temperature of the reaction system would immediately rise after the solution was dropwise added, and was required to be controlled to below 5°C. The dropwise addition lasted for about 30 minutes, and the reaction was performed under stirring at 5°C for 30 minutes and at room temperature for 2 h. After the reaction was monitored by thin layer chromatography (TLC) to be completed, the reaction mixture was washed with 0.1 mol/L of sodium hydroxide solution, washed with saturated sodium chloride aqueous solution, washed twice with deionized water, and subjected to rotary evaporation to remove the solvent, and drying in a vacuum oven for 24 h to obtain a viscous light-yellow sample for direct use.

When $R_1$ was methyl, the product was a methanesulfonyl-protected compound with 98% yield. MS ($C_{17}H_{25}NO_5S_2$): m/e: 387.51.

Experimental results: 388.52 ($M+H^+$); $^1H$ NMR ($CDCl_3$, 400MHz): 8.44 (d, 2H); 7.28 (d, 2H); 4.32 (t, 2H, $CH_2$); 3.63 (t, 4H, $CH_2$); 3.28 (t, 2H, $CH_2$); 2.96 (s, 3H, $CH_3$); 2.50 (t, 4H, $CH_2$); and 1.24 (t, 6H, $CH_3$).

When $R_1$ was p-methylphenyl, the product was a p-toluenesulfonyl-protected compound with 98.5% yield. MS ($C_{23}H_{29}NO_5S_2$): m/e: 463.61; and experimental result: 464.62 ($M+H^+$).

Methanesulfonyl-protected product was used to prepare the double-bond-containing α-aminoketone photoinitiator A.

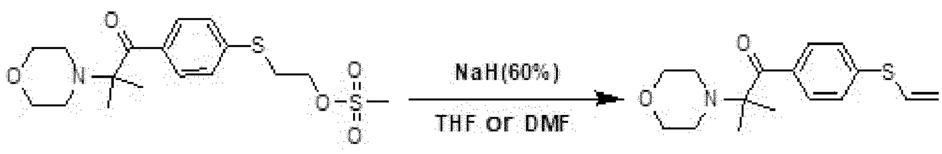

To a 500 mL three-neck flask were sequentially added 0.1 mol of sodium hydride, 200 mL of anhydrous tetrahydrofuran (THF) or Dimethyl Formamide (DMF), to which 38.7 g of the methanesulfonyl-protected compound (0.1 mol) dissolved in 100 mL of THF or DMF were dropwise added in the nitrogen atmosphere at room temperature and reacted under stirring for 5 h. After the reaction was monitored by TLC to be completed, the reaction mixture was concentrated under reduced pressure or poured into 1000 mL water, extracted with ethyl acetate, washed with saturated salt water and deionized water, dried with anhydrous sodium sulfate, and evaporated and recrystallized with ethanol/water to obtain a light-yellow powder.

The yield of the double-bond-containing α-aminoketone photoinitiator A was 78.5%. MS ($C_{16}H_{21}NO_2S$): m/e: 291.13; and experimental result: 292.14 ($M+H^+$).

The $^1H$ NMR spectrum of the photoinitiator A were shown in FIG. 6, where the four hydrogens on the benzene ring at 8.52 and 7.35 ppm; three hydrogens on the double bond at 6.57 and 5.57 ppm; eight hydrogens on the morpholine ring at 3.69 and 2.57 ppm; and the signal peaks of six hydrogens on two methyl groups at 1.31 ppm, which indicated that the double-bond-containing α-aminoketone photoinitiator had been successfully synthesized.

## Example 3 Preparation of photoinitiator 1 by the thiol-ene click reaction

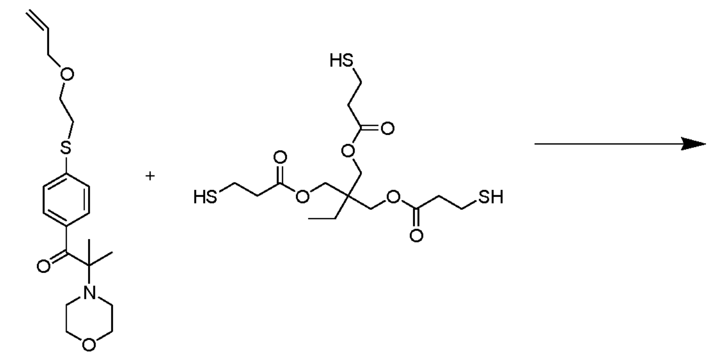

**(1)**

To a 100 mL three-neck flask were sequentially added 10.47 g (30 mmol) of double-bond-containing α-aminoketone photoinitiator A, 3.98 g (10 mmol) of polythiol compound B, and 0.145 g of azobisisobutyronitrile to obtain a reaction mixture. The reaction mixture was vacuumized, filled with nitrogen gas for three cycles, placed in a 50°C oil bath, stirred for 30 min, heated and reacted at 70°C for 2 h, and heated and reacted at 90°C for 1 h. After the absorption peak of thiol at 2570 $cm^{-1}$ was absent by monitoring by infrared spectroscopy, and raw materials for thin-plate chromatography were used up, indicating completion of the reaction. The target product photoinitiator 1 was prepared from the double-bond-containing α-aminoketone photoinitiator A and the polythiol compound B by the thiol-ene click reaction.

After the reaction was completed, the reaction mixture could be directly used to $^1H$ NMR test without purifying the reaction mixture. The $^1H$ NMR spectrum was shown in FIG. 1. It could be seen that except for a few fragment peaks of azobisisobutyronitrile (AIBN), the rest were product peaks, showing that the thiol-ene click reaction could convert almost 100% of the reactants into the target product.

$^1H$ NMR($CDCl_3$, 400MHz): 8.50 (d, J = 8.0 Hz, 6H, Ph); 7.28 (d, J = 8.0 Hz, 6H, Ph); 4.06 (s, 6H, $CH_2$); 3.70 (m, 18H, $CH_2$); 3.55 (t, J = 6.0 Hz, 6H, $CH_2$); 3.20 (t, J = 6.6 Hz, 6H, $CH_2$); 2.76 (t, J = 7.4 Hz, 6H, $CH_2$); 2.59 (m, 24H, $CH_2$); 1.86 (m, 6H, $CH_2$); 1.48 (q, 2H, $CH_2$); 1.33(s, 18H, $CH_3$); and 0.89 (t, 3H, $CH_3$).

## Example 4 Preparation of photoinitiator 2 by the thiol-ene click reaction

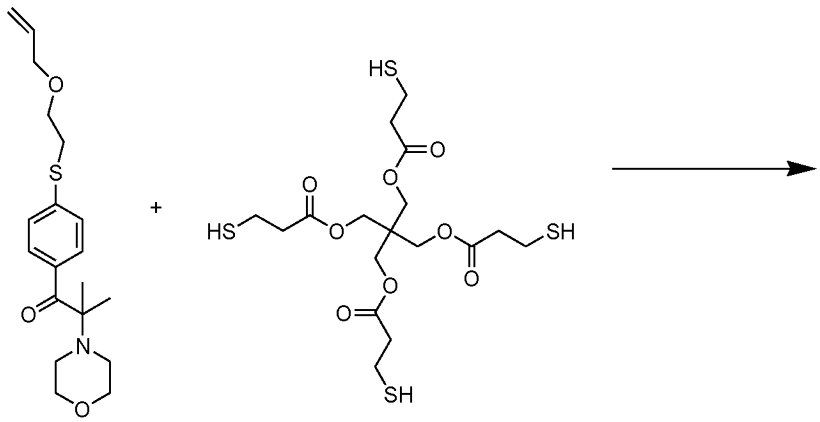

**(2)**

To a 100 mL three-neck flask were sequentially added 13.96 g (40 mmol) of double-bond-containing α-aminoketone photoinitiator A, 4.98 g (10 mmol) of tetra-thiol, and 0.190 g of azobisisobutyronitrile to obtain a reaction mixture. The reaction mixture was evacuated, filled with nitrogen gas for three cycles, placed in a 50°C oil bath, stirred for 30 min, heated and reacted at 70°C for 2 h, and heated and reacted at 90°C for 1 h. After the absorption peak of thiol at 2570 $cm^{-1}$ was absent by monitoring by infrared spectroscopy, and raw materials for thin-plate chromatography were used up, indicating completion of the reaction. The target product photoinitiator 2 was prepared from the double-bond-containing α-aminoketone photoinitiator A and the tetra-thiol by the thiol-ene click reaction.

After the reaction was completed, the reaction mixture could be directly used to $^1H$ NMR test without purifying the reaction mixture. The $^1H$ NMR spectrum was shown in FIG. 2. It could be seen that except for a few fragment peaks of AIBN, the rest were product peaks, showing that the tetrafunctional tetra-thiol could react almost 100%. The target product had good symmetry, there was no signal of the ethyl group of the trisubstituted target product, and the number of other peaks was a multiple of 8 and 8, which showed the purity of the reaction.

$^1H$ NMR ($CDCl_3$, 400MHz): 8.50 (d, J = 8.0 Hz, 8H, Ph); 7.30 (d, J = 8.0 Hz, 8H, Ph); 4.16 (s, 8H, $CH_2$); 3.70 (d, 24H, $CH_2$); 3.57 (t, J = 6.0 Hz, 8H, $CH_2$); 3.20 (t, J = 6.6 Hz, 8H, $CH_2$); 2.75 (t, J = 7.4 Hz, 8H, $CH_2$); 2.60 (t, 8H, $CH_2$); 2.57 (d, 24H, $CH_2$); 1.84 (m, 8H, $CH_2$); and 1.31(s, 24H, $CH_3$).

## Example 5 Preparation of photoinitiator 3, photoinitiator 4, or photoinitiator 5 from different polythiol compounds B and corresponding α-aminoketone-containing double-bonded micromolecular photoinitiators A

The three reactions have the same steps as those of Example 3 or Example 4.

The final products of three reactions were tested by $^1H$ NMR respectively. It could be seen from the NMR hydrogen spectra that the conversion of polythiol compound B and double-bond-containing α-aminoketone photoinitiator A in the three reactions was almost 100%. The molar ratio of a thiol group in the polythiol compound B to a carbon-carbon double bond in the double-bond-containing α-aminoketone photoinitiator A was 1:1. The target product photoinitiators could be prepared from the double-bond-containing α-aminoketone photoinitiator A and the polythiol compound B by the thiol-ene click reaction. The conversion of the double-bond-containing α-aminoketone photoinitiator A and the polythiol compound B was almost 100%.

## Performance test

The following experiments were conducted separately to test performance of the target products obtained in the above Examples.

## Experimental Example 1

The photoinitiators 1-5 prepared in the Examples and photoinitiator 907 were tested for light curing in monomers, respectively. According to the following mass ratio, the test solutions were prepared by mixing the photoinitiators 1-5 or the photoinitiator 907, respectively.

Bifunctional monomer (TPGDA): 97%;
Photoinitiator (1, 2, 3, 4, 5 or 907): 2%;
Leveling agent: 0.5%; and
Defoamer: 0.5%.

The test solutions were respectively coated on the cardboard to form a coating of about 30~35μm. The LED light source (LED surface light source with width of 3 cm and length of 80 cm) with a unit power of 2000 mW/$cm^2$ and an emission wavelength of 365 nm, produced by Guangzhou He Guang Tong Sheng Company, was used as the excitation light source and placed on a variable speed conveyor belt. Repeated scratching with nails without imprinting was the standard for the completion of photopolymerization curing.

The test results showed that the curing speed of the photoinitiator 1, photoinitiator 2, photoinitiator 3, photoinitiator 4, photoinitiator 5 and photoinitiator 907 in the monomer did not show any difference.

**Experimental Example 2**

The photoinitiator 1 was used for light curing test in the coating formulations.

According to the following mass percentages, the curing solutions were prepared by the photoinitiator 1 and photoinitiator 907, respectively.

Epoxy acrylate (621A-80): 15%;
Aromatic urethane acrylate (6146-100): 24%;
Aliphatic polyurethane acrylate (6145-100): 20%;
Trifunctional monomer (TMPTA): 23%;
Difunctional monomer (HDDA): 3%;
Bifunctional monomer (TPGDA): 12%;
Photoinitiator (1 or 907): 2.5%; and
Leveling agent: 0.5%.

The photoinitiator 1 and photoinitiator 907 curing solution were coated on the substrate to form a coating with thickness of about 20~25μm. The RW-UVA201-20 mercury lamp curing machine produced by Shenzhen Runwo Mechanical & Electrical Co., Ltd. was used as the excitation light source with light intensity of 220mW/$cm^2$, placed on a conveyor belt (speed of about 8 m/min), and cured 3 times, to test the pencil hardness, adhesive force, flexibility, and glossiness of the cured coating. In addition, 18.00g of the curing solution was placed in a centrifuge tube and placed in a 60°C oven for 7 days, to obtain the change in viscosity of the system before and after thermal storage. The results were shown in Table 1.

Table 1 Test results of coating properties

| Performance | Photoinitiator 907 system | Photoinitiator 1 system |
|---|---|---|
| Pencil hardness | 3H | 3H |
| Adhesive force (ABS board) | Grade 1 | Grade 1 |
| Flexibility (mm) | >7.5 | >7.5 |
| Yellowing (Δb) | 1.98 (after cured 4 times) | 1.02 (after cured 4 times) |
| | 6.77 (after cured 40 times) | 3.71 (after cured 40 times) |
| Glossiness (GU) | 94.6±0.14 | 92.3±0.12 |
| Change in viscosity | No significant change | No significant change |

The test results showed that photoinitiator 1 and photoinitiator 907 showed no difference in curing speed in the coating formulation, but the anti-yellowing performance of the photoinitiator 1 was significantly better than that of the photoinitiator 907.

**Experimental Example 3**

The photoinitiators 1-5 were used for migration test of light-curing product, respectively.

1% photoinitiators 1-5 or photoinitiator 907 trimethylolpropane triacrylate solutions were prepared, respectively. The photoinitiators 1-5 or photoinitiator 907 trimethylolpropane triacrylate solutions were respectively coated on a slide, to form a film with thickness of 30-35 μm. The RW-UVA201-20 mercury lamp curing machine produced by Shenzhen Runwo Mechanical & Electrical Co., Ltd. was used as the excitation light source with light intensity of 220 mW/$cm^2$, placed on the conveyor belt (speed of about 30 m/s), and cured 5 times. The cured films were peeled and cut. 100 mg film fragments were soaked in 20 mL acetonitrile for 24 h, and the supernatant was tested 3 times for the UV-visible absorption spectrum. The

absorbance of the maximum absorption peaks of the photoinitiators 1-5 and photoinitiator 907 at 307 nm was recorded.

The relative migration rate of the photoinitiators 1-5 and 907 could be calculated from the following formula.

$$c = A/(\varepsilon \bullet b);$$

and

$$R = c(I)/c(907)*100.$$

In the above formulas, c is concentration of the photoinitiator in the extract solution in mol/L; A is the absorbance; ε is molar absorbance coefficient of the photoinitiator at 307 nm in L/(mol•cm); b is thickness of the sample cell in cm; c(I) is concentration of the photoinitiator I in the extract solution (the photoinitiator I is one of photoinitiators 1-5); c(907) is concentration of photoinitiator 907 in the extract solution; R is a relative mobility of the photoinitiator I in contrast to the photoinitiator 907. The absorbance of the solution containing photoinitiator 907 was defined as 100, and the relative migration rate R of the photopolymerization system initiated by other molecules were shown in Table 1.

Table 1 Mobility test results

| Formulat ion | Photoiniti ator 907 | Photoiniti ator 1 | Photoiniti ator 2 | Photoiniti ator 3 | Photoiniti ator 4 | Photoiniti ator 5 |
|---|---|---|---|---|---|---|
| Migratio n rate R | 100 | 14 | 12 | 13 | 13 | 12 |

The test results showed that compared with the photoinitiator 907, the migration rate of the photoinitiators 1-5 decreased significantly, and it could be concluded that compared with the photoinitiator 907, the migration rate of the photoinitiator I decreased significantly.

## Experimental Example 4

The photoinitiator 1 was used for migration test of light-curing products in the coating formulations.

Four curing solutions were prepared according to the following mass percentages.

Polyester acrylate (6342): 20%; and
Trifunctional monomer (TMPTA): 80%.

The photoinitiator 1 and photoinitiator 907 were added to each of the four curing solutions, respectively. The addition amount of the photoinitiator 1 or photoinitiator 907 was 3% or 6% of total mass of the curing solution, respectively.

The above different concentrations of the photoinitiator 1 and photoinitiator 907 solution were respectively coated on the PET film to form 20~25μm coating. The RW-UVA201-20 mercury lamp curing machine produced by Shenzhen Runwo Mechanical & Electrical Co., Ltd. was used as the excitation light source with light intensity of 220mW/$cm^2$, placed on the conveyor belt (speed of about 8 m/min), and cured 3 times. The cured films were peeled and cut. 0.50 g film fragments were soaked in 10 mL ethanol at 40°C for 10 days, and the supernatant was collected and tested by a UV spectrophotometer to analyze the migration rate of the photoinitiators. The average value of two test results was taken. The results were shown in Table 2.

Table 2 Mobility test results

| Initiator content | Photoinitiator 907-3% | Photoinitiator 907-6% | Photoinitiator I -3% | Photoinitiator I -6% |
|---|---|---|---|---|
| Migration rate (mg/mL) | 0.468 | 0.907 | 0.06 | 0.094 |

The test results showed that the migration rate of the photoinitiator 1 was less than 1/8 that of the photoinitiator 907, showing a significant decrease.

## Experimental Example 5

The photoinitiator 1 was used for testing curing odor.

The photoinitiator 1 and photoinitiator 907 were prepared in solutions according to the following mass percentages.

Trifunctional monomer (TMPTA): 64.9%;
Difunctional monomer (HDDA): 30%;
Leveling agent: 0.6%;
Defoamer: 0.5%; and
Photoinitiator (1 or 907): 4%

The photoinitiator 1 and photoinitiator 907 solution were coated on the PET film to form a coating with thickness of about 40~45μm and coating area of about 8 × 20 cm. The RW-UVA201-20 mercury lamp curing machine produced by Shenzhen Runwo Mechanical & Electrical Co., Ltd. was used as the excitation light source with light intensity of 220 $mW/cm^2$, placed on the conveyor belt (speed of about 8m/min), and cured once. In the curing process, three testers at the same time smelled whether there is a pungent odor. The odor was evaluated according to the following levels:
Level 0-no pungent odor; Level 1-slight pungent odor; Level 2-medium pungent odor; Level 3-medium-high pungent odor; and Level 4-high pungent odor.

Each formulation was tested twice, and the odor evaluation level which occurred most was taken as the final result. The test results were shown in Table 3.

Table 3 Curing odor test results

| Photoinitiator | Odor level |
|---|---|
| Photoinitiator 907 system | Level 3 |
| Photoinitiator 1 system | Level 1 |

The test results showed that compared with the photoinitiator 907, the odor level of the photoinitiator I with the molecular weight of 1000 or more was significantly less.

## Experimental Example 6

The photoinitiator 1 and photoinitiator 907 were tested for photo-curing performance in TMPTA.

The photoinitiability of the photoinitiator 1 was compared with that of the photoinitiator 907. FIGS. 3 and 4 showed the photopolymerization kinetic curves and Photo-DSC polymerization exotherm curves of TMPTA initiated by the photoinitiator 1 and the photoinitiator 907 in the same weight and in the same number of moles, respectively.

As shown in FIG. 4, when the photoinitiator 1 and the photoinitiator 907 were in the same number of moles, the exotherm rate of polymerization and the final conversion rate of the photoinitiator 1 and photoinitiator 907 were basically the same.

As shown in FIG. 3, when the photoinitiator 1 and photoinitiator 907 were in the same weight, the exotherm rate of the photoinitiator 1 reached highest after 8 s, and the maximum rate was 34.67 mW/mg, which was slightly lower than that of the photoinitiator 907. With the extension of the light time, the total exotherm of the double bond polymerization of the resin initiated by the photoinitiator 907 was higher, and the overall conversion rate was slightly higher. Referring to FIG. 4, it could be concluded that when the macromolecular photoinitiator 1 and the photoinitiator 907 with the same weight initiated TMPTA, the molar concentration of the photoinitiator 1 in the curing system was lower, resulting in lower exotherm and final conversion rate. This difference could be narrowed by adding the photoinitiator 1 with same number of moles. In combination with FIGS. 3 and 4, it could be learned that photoinitiator 1 had almost the same efficiency as that of the photoinitiator 907. The photoinitiator 1 was potential in industrialization.

## Experimental Example 7

The photoinitiator 1 was tested for antioxidant-inhibition test in coating formulations.

Two curing solutions were prepared according to the following mass percentages.

Polyester acrylate (6342): 60%; and
Trifunctional monomer (TMPTA): 40%.

The photoinitiator 1 and photoinitiator 907 in the same number of moles were respectively added to each of the 2 curing solutions. The addition amount of the photoinitiator 907 was 3% of the total mass of the curing solution.

The curing solutions were respectively coated on a PET film to form a coating with thickness of 20-25 μm, placed

under 365 nm LED surface light source with light intensity of 700mW/$cm^2$ which was produced by YIWATA (Shanghai) Precision Optoelectronics Co., Ltd., placed on the conveyor belt (speed of 8 m/min), and cured 3 times. Then the cured sample was cut into 6×6cm square, baked at 40°C for 15 min, weighed, wiped with skimmed cotton dipped in alcohol to remove the uncured part of the coating surface, dried in the oven at 40°C for 30 min, weighed again, and recorded the difference between the two weighing results, to calculate the uncured thickness. The average value of 3 test results was taken. The results were shown in Table 4.

Table 4 Antioxidant-inhibition test results

| Photoinitiator | 907 | I |
|---|---|---|
| Uncured surface thickness | 1.89±0.26 | 1.71±0.07 |
| (μm) | | |

The test results showed that photoinitiator I had a better antioxidant-inhibition effect than photoinitiator 907. Compared the structural formulas of the photoinitiator I and photoinitiator 907, it was found that photoinitiator I could improve the antioxidant-inhibition effect of photoinitiators in the coating due to the introduction of thioether.

**Experimental Example 8**

The photoinitiator 1 was tested for refractive index in coating formulations.
Two curing solutions were prepared according to the following mass percentages.

Polyester acrylate (6342): 60%; and
Trifunctional monomer (TMPTA): 40%.

The photoinitiator 1 and photoinitiator 907 in the same number of moles were respectively added into the curing solution. The addition amount of photoinitiator 907 added was 3% of the total mass of the curing solution.
The photoinitiator 1 and photoinitiator 907 curing solutions were respectively tested for refractive index by an Abbe refractometer. The average value of two test results was taken. The results were shown in Table 5.

Table 5 Refractive index test results

| Photoinitiator | 907 | 1 |
|---|---|---|
| Refractive index $n_D$ (13°C) | 1.4930 | 1.4946 |

The test results showed that the refractive index of the curing system containing the photoinitiator 1 was better than that of the curing system containing the photoinitiator 907.

**Experimental Example 9**

The photoinitiator 1 and photoinitiator 907 were tested for DSC thermal stability.
In order to compare the thermal stability of the photoinitiator 907 and the photoinitiator 1, the difference in thermal stability of the two photoinitiators was tested under nitrogen protection at a temperature rate of 10°C/min, with a nitrogen flow rate of 50 mL/min. As shown in FIG. 5, compared with the photoinitiator 907, the thermal stability of the photoinitiator 1 had significantly improved, there was no significant thermal effect within 300°C.
The experimental analysis was conducted as follows.
From Experimental Example 1, Experimental Example 2, Experimental Example 6, Experimental Example 8, and Experimental Example 9, it could be obtained that there was no significant difference between the curing speed of the photoinitiator I and that of the photoinitiator 907. The anti-yellowing property of the photoinitiator I was significantly better than that of the photoinitiator 907. When the photoinitiator I and the photoinitiator 907 in the same number of moles were cured in the initiating curing system, the polymerization exothermic rate and the final conversion rate of the photoinitiator I and the photoinitiator 907 were basically the same. The photoinitiator I had better heat stability than the photoinitiator 907. The refractive index of the curing system containing photoinitiator I was relatively better than that of the curing system containing photoinitiator 907.
From Experimental Example 3, Experimental Example 4, and Experimental Example 5, it could be obtained that the molecular weight of the photoinitiator I was larger than that of the photoinitiator 907. The migration rate of photoinitiator I was significantly lower than that of the photoinitiator 907 in the cured products using the monomer formulation or the

coating formulation. When curing, compared with the photoinitiator 907, the photoinitiator I had less irritating odor. Furthermore, the photoinitiator I was suitable for the application areas with high biological toxicity requirements.

As shown in Experimental Example 7, the photoinitiator I had a better antioxidant-inhibition effect than photoinitiator 907 in the coating. The photoinitiator I had a faster surface drying speed of the cured product when triggering the curing system to cure.

In summary, the photoinitiator I had better performance than the photoinitiator 907, so that the photoinitiator I could replace the photoinitiator 907, and had a wider application range than the photoinitiator 907.

## Claims

1. An α-aminoketone-containing polyfunctionalized macromolecular photoinitiator, wherein the α-aminoketone-containing polyfunctionalized macromolecular photoinitiator is prepared from a double-bond-containing α-aminoketone photoinitiator A and a polythiol compound B through thiol-ene click reaction.

2. The α-aminoketone-containing polyfunctionalized macromolecular photoinitiator of claim 1, wherein the double-bond-containing α-aminoketone photoinitiator A is expressed as:

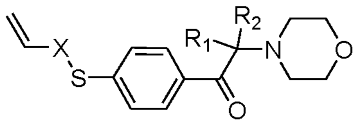
;

wherein $R_1$ and $R_2$ are each independently $C_1$-$C_{18}$ alkyl, $-(CH_2)_r$-$C_3$-$C_6$ cycloalkyl group or $-(CH_2)_r$-phenyl, wherein r is 0, 1, 2, 3, or 4; and

X is an unsubstituted alkyl, a substituted alkyl with at least one $-CH_2-$ each independently substituted with -O-, -CO-, -COO-, -OCO-, or $-O-CH_2-CH(OH)-CH_2-O-$, or absent.

3. The α-aminoketone-containing polyfunctionalized macromolecular photoinitiator of claim 2, wherein both $R_1$ and $R_2$ are methyl when X is absent.

4. The α-aminoketone-containing polyfunctionalized macromolecular photoinitiator of claim 1, wherein the polythiol compound B is a trithiol or a tetrathiol.

5. The α-aminoketone-containing polyfunctionalized macromolecular photoinitiator of claim 4, wherein the polythiol compound B is selected from the group consisting of:

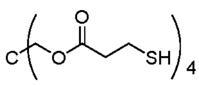
B1
,
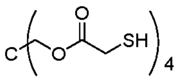
B2
,
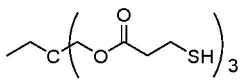
B3
,
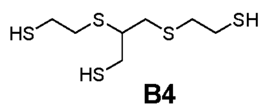
B4
,
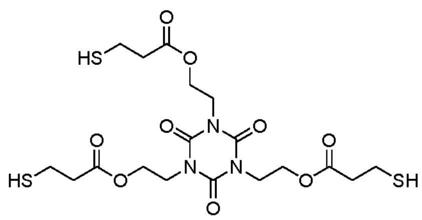
B5
,
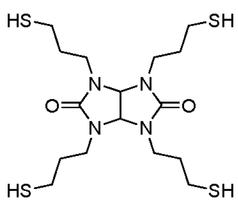
B6
,

and a combination thereof.

6. The α-aminoketone-containing polyfunctionalized macromolecular photoinitiator of claim 1, wherein the α-aminoketone-containing polyfunctionalized macromolecular photoinitiator is selected from the group consisting of:

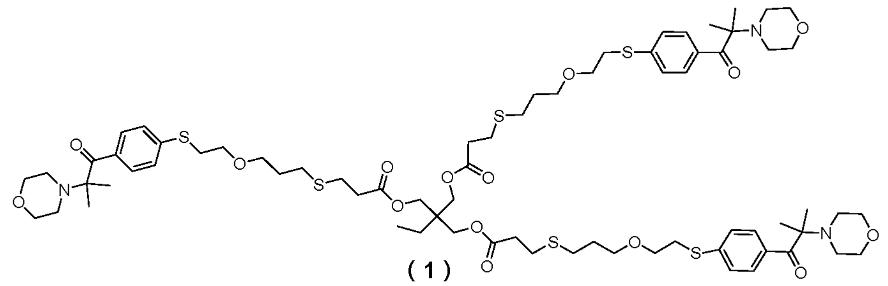

( 1 )

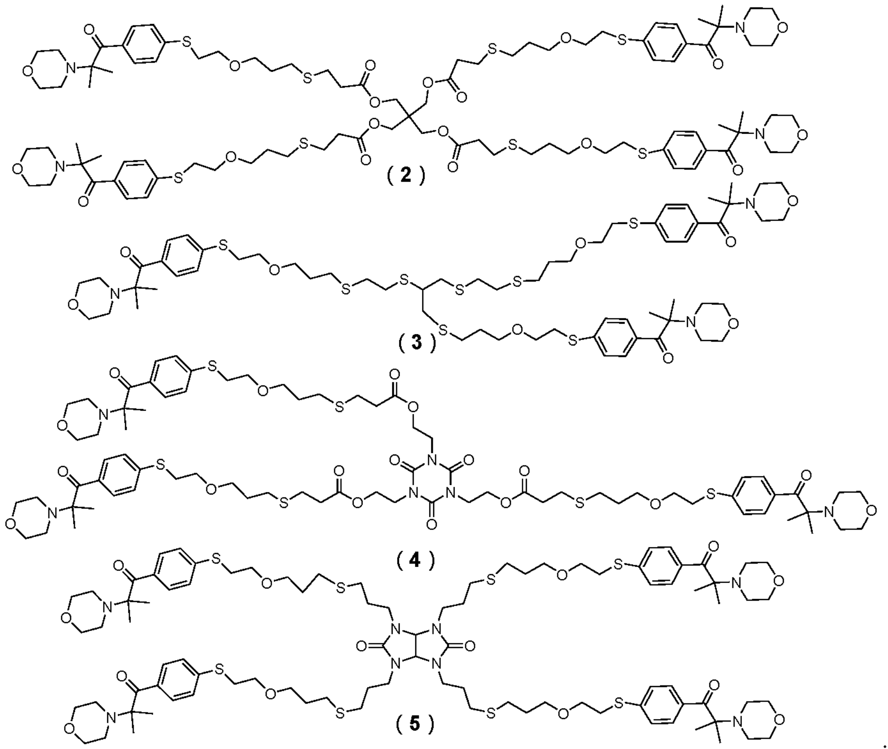

( 2 )

( 3 )

( 4 )

( 5 )

.

7. A method for preparing the α-aminoketone-containing polyfunctionalized macromolecular photoinitiator of claim 1, comprising:

(a) mixing the double-bond-containing α-aminoketone photoinitiator A and the polythiol compound B to obtain a mixture; wherein a molar ratio of a carbon-carbon double bond in the double-bond-containing α-aminoketone photoinitiator A to a thiol group in the polythiol compound B is 1: 1;
(b) adding an azodiisobutyronitrile (AIBN) thermal initiator or a benzoyl peroxide thermal initiator into the mixture followed by stirring under nitrogen atmosphere and reaction at 60~100°C for 2~12 h to obtain a reaction solution, wherein the AIBN thermal initiator or the benzoyl peroxide thermal initiator is 0.5%-3% of a total weight of the mixture, and whether the reaction is completed or not is determined by monitoring an absorption peak of thiol by infrared spectroscopy, and when the absorption peak of thiol is absent, it indicates completion of the reaction; and
(c) subjecting the reaction solution to recrystallization to obtain the α-aminoketone-containing polyfunctionalized macromolecular photoinitiator.

8. A photoinitiator composition for radiation curing, comprising:
the α-aminoketone-containing polyfunctionalized macromolecular photoinitiator of claim 1.

9. The photoinitiator composition of claim 8, wherein an excitation light source of the photoinitiator composition is ultraviolet light, visible light, or a combination thereof.

10. The photoinitiator composition of claim 9, wherein the photoinitiator composition comprises 0.01~30 parts by weight of the α-aminoketone-containing polyfunctionalized macromolecular photoinitiator and 100 parts by weight of a double

bond-containing unsaturated compound.

**11.** The photoinitiator composition of claim 10, wherein the photoinitiator composition comprises 0.5~10 parts by weight of the α-aminoketone-containing polyfunctionalized macromolecular photoinitiator and 100 parts by weight of the double bond-containing unsaturated compound.

**12.** The photoinitiator composition of claim 11, wherein the double bond-containing unsaturated compound is a compound or mixture whose double bonds are cross-linked by free-radical polymerization reaction; and
the double bond-containing unsaturated compound is selected from the group consisting of a monomer, an oligomer, a prepolymer, a combination thereof, a copolymer thereof and an aqueous dispersion thereof.

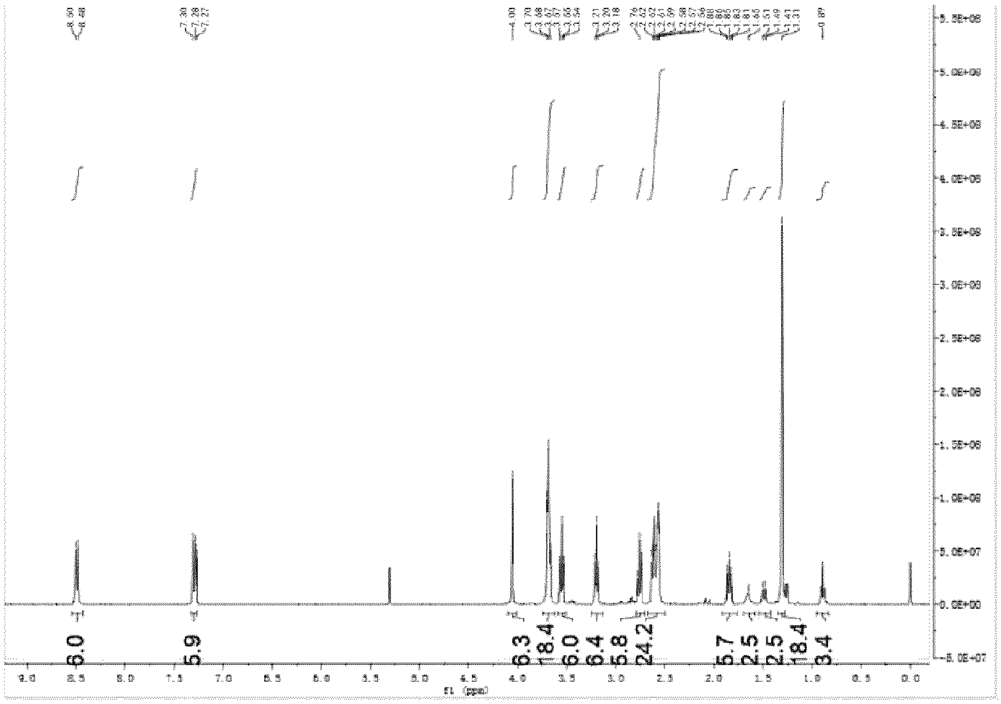

FIG.1

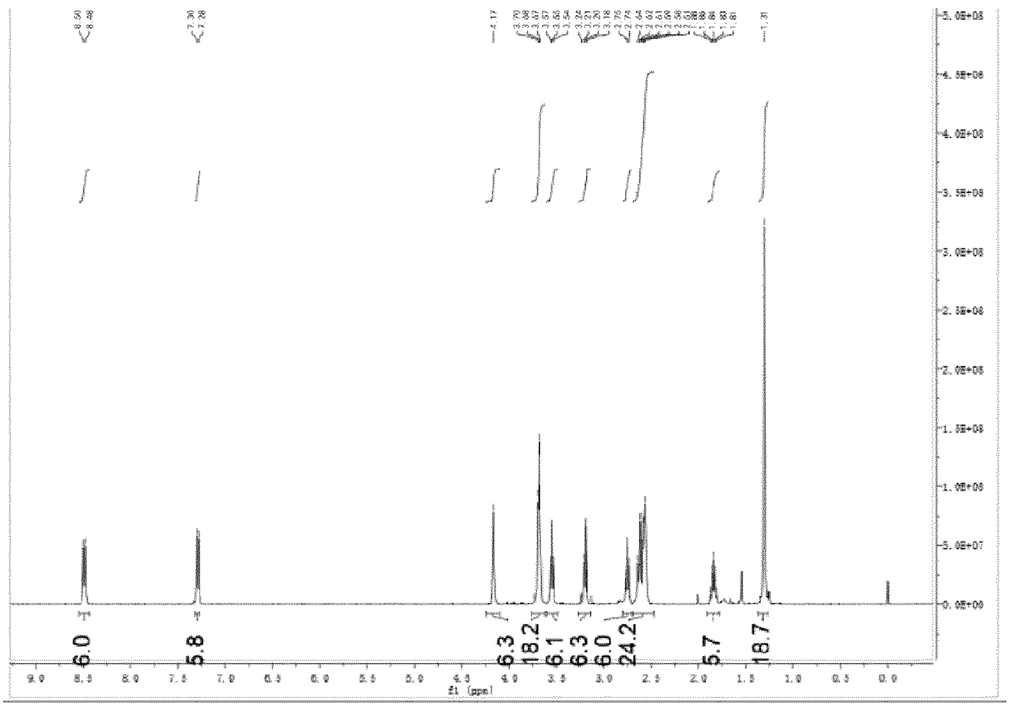

FIG.2

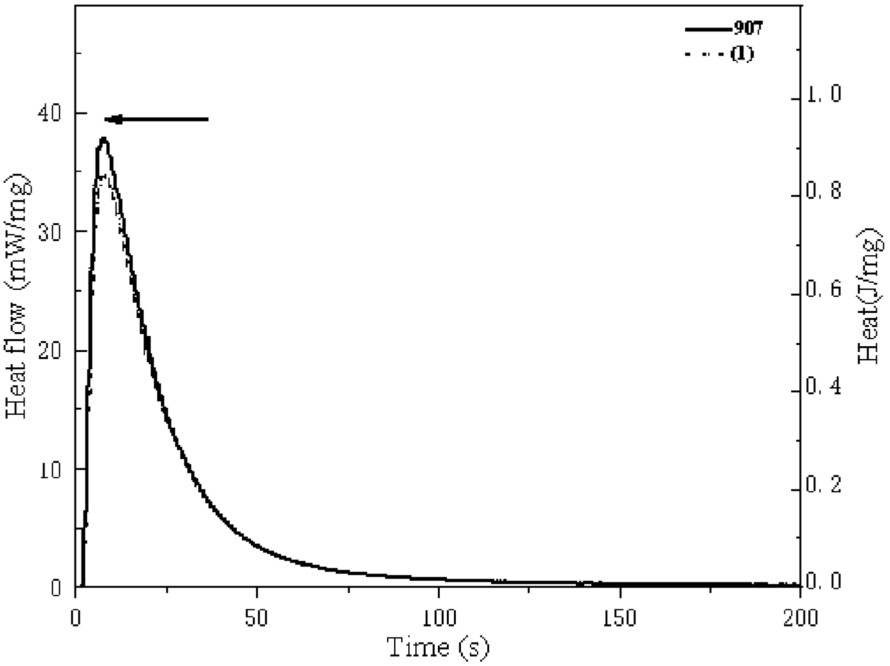

907
(1)
Heat flow (mW/mg)
Heat(J/mg)
Time (s)
0
10
20
30
40
0
50
100
150
200
0.0
0.2
0.4
0.6
0.8
1.0


FIG.3

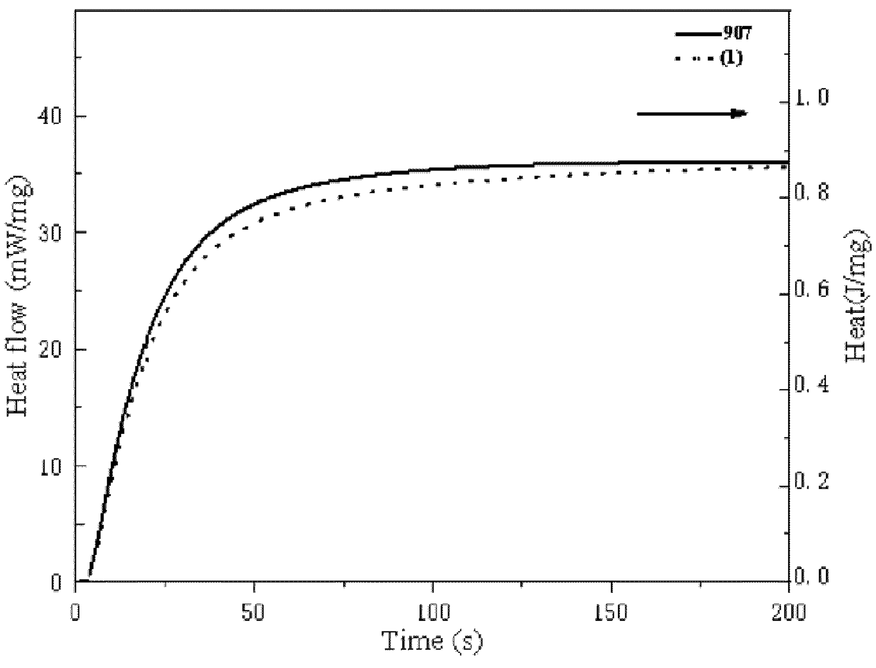

907
(1)
Heat flow (mW/mg)
Heat(J/mg)
Time (s)
0
10
20
30
40
0
50
100
150
200
0.0
0.2
0.4
0.6
0.8
1.0


FIG.4

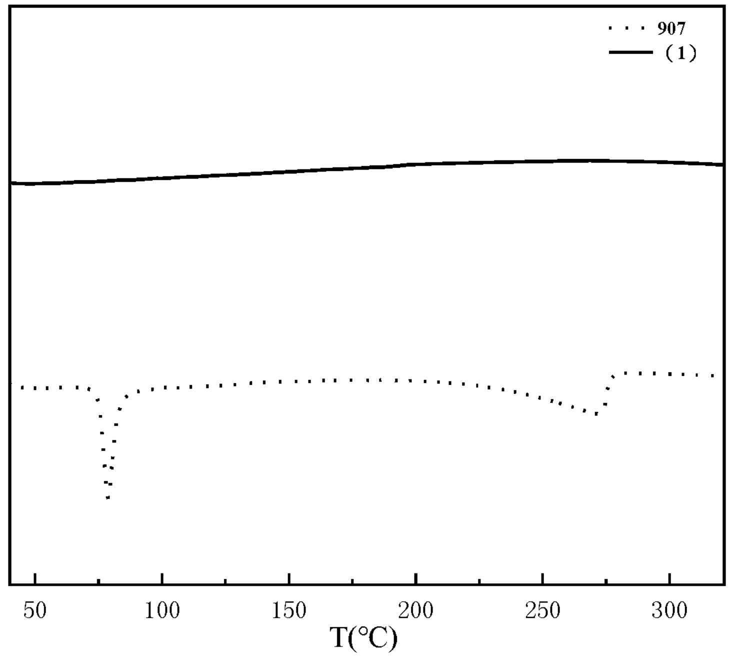


FIG.5

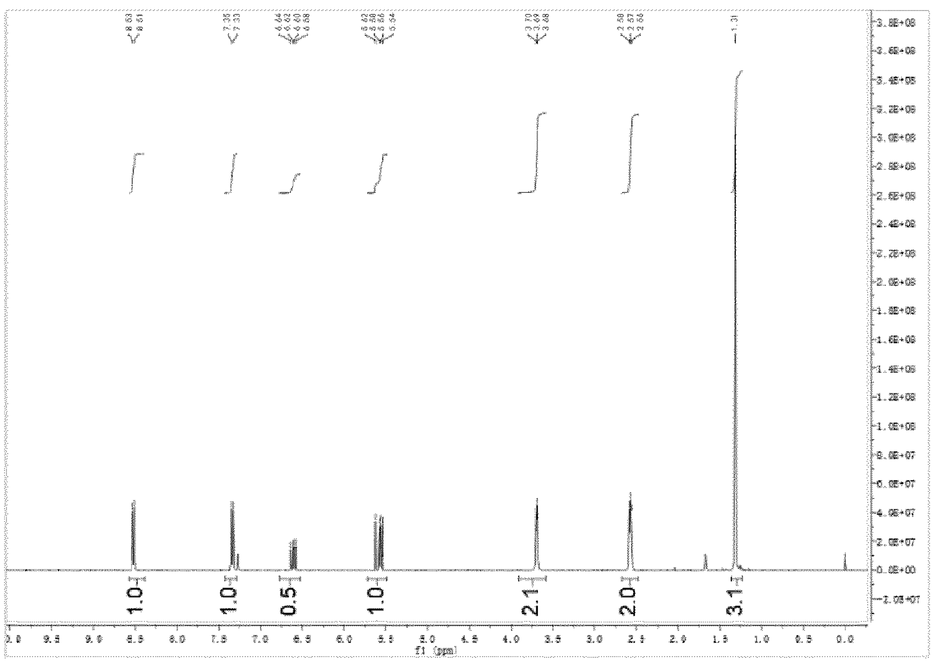


FIG. 6

## INTERNATIONAL SEARCH REPORT

International application No.
**PCT/CN2023/079014**

**A. CLASSIFICATION OF SUBJECT MATTER**

C08F2/48(2006.01)i;C07D295/104(2006.01)i;C07D487/04(2006.01)i;C07D251/30(2006.01)i;C09D4/02(2006.01)i;C09D175/14(2006.01)i;C09D167/06(2006.01)i

According to International Patent Classification (IPC) or to both national classification and IPC

**B. FIELDS SEARCHED**

Minimum documentation searched (classification system followed by classification symbols)

IPC：C08F,C07D,C09D

Documentation searched other than minimum documentation to the extent that such documents are included in the fields searched

Electronic data base consulted during the international search (name of data base and, where practicable, search terms used)

CNABS, VEN, CNTXT, ENTXT: 氨基酮, 胺基酮, 光引发剂, 硫醇, amido, ketone, amine, photo, initiator, initiating, mercaptan, thiol, thioalcohol,

**C. DOCUMENTS CONSIDERED TO BE RELEVANT**

| Category* | Citation of document, with indication, where appropriate, of the relevant passages | Relevant to claim No. |
|---|---|---|
| PX | CN 114591452 A (INNER MONGOLIA YANGTZE NEW MATERIALS CO., LTD.) 07 June 2022 (2022-06-07) entire document | 1-12 |
| A | CN 101974112 A (NANTONG XINYU CHEMICAL CO., LTD.) 16 February 2011 (2011-02-16) entire document | 1-12 |
| A | WO 2006034966 A1 (CIBA SPECIALTY CHEMICALS HOLDING INC. et al.) 06 April 2006 (2006-04-06) entire document | 1-12 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 May 2023** | **11 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/079014**

| Patent document cited in search report | | | Publication date (day/month/year) | Patent family member(s) | | | Publication date (day/month/year) |
|---|---|---|---|---|---|---|---|
| CN | 114591452 | A | 07 June 2022 | None | | | |
| CN | 101974112 | A | 16 February 2011 | None | | | |
| WO | 2006034966 | A1 | 06 April 2006 | DE | 602005008626 | D1 | 11 September 2008 |
| | | | | JP | 2008514671 | A | 08 May 2008 |
| | | | | AT | 402920 | T | 15 August 2008 |
| | | | | US | 2011130482 | A1 | 02 June 2011 |
| | | | | TW | 200616937 | A | 01 June 2006 |
| | | | | EP | 1799638 | A1 | 27 June 2007 |
| | | | | EP | 1799638 | B1 | 30 July 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023079014 W
- CN 202210246991